# EUROPEAN PATENT APPLICATION

(11) **EP 4 678 088 A1**
(43) Date of publication of application: **14.01.2026**
(21) Application number: 25167891.8
(22) Date of filing: 02.04.2025
(51) Int. Cl.: A61B 1/00, A61B 1/04, A61B 1/06

(54) **FILTER DEVICE**

(30) Priority: 09.07.2024 JP 2024110540
(71) Applicant: Olympus Medical Systems Corp., Tokyo 192-8507 (JP)
(72) Inventor: KITAMURA, Ojiro, Tokyo, 1928507 (JP)
(74) Representative: Gunzelmann, Rainer

(57) **Abstract**

A filter device 30 includes a filter 31 configured to attenuate light with a predetermined wavelength from emitted light emitted through a cover glass 13 of an eyepiece portion 12 of an endoscope 10 along an optical axis O, a frame 32 configured to hold the filter 31, and a connector 33 configured to hold the frame 32, and attach the frame 32 to a camera head 20 including an image pickup device 21 configured to receive light passed through the filter 31. The connector 33 includes an elastic region 33ER that presses the filter 31 or the frame 32 to an outer circumference of an effective optical path EA of the cover glass 13 or to a circumference of the cover glass 13, in a state where the camera head 20 is attached to the eyepiece portion 12.

## Description

### CROSS REFERENCE TO RELATED APPLICATION

This application claims the benefit of Japanese Application No.2024-110540 filed in Japan on July 9, 2024, the contents of which are incorporated herein by this reference.

### BACKGROUND OF THE INVENTION

### 1. Field of the Invention

The present invention relates to a filter device disposed between an eyepiece portion of an endoscope and a camera head.

### 2. Description of the Related Art

International Publication No. 2016/057483 discloses a camera head that is detachably attached to an eyepiece portion of an endoscope configured to acquire an object image, and picks up the object image emitted from the eyepiece portion.

A filter device is disposed between the eyepiece portion of the endoscope and the camera head, to block or attenuate laser wavelength light from emitted light of the endoscope that performs a laser surgery.

In addition, in an endoscope that performs a fluorescent observation by irradiating a lesion site with excitation light and detecting fluorescence from the lesion site, a filter device that blocks or attenuates the excitation light is also essential.

However, if a filter of the filter device is not arranged parallel to a cover glass that constitutes an emission surface of the eyepiece portion, reflected light occurs between the cover glass and the filter, and so-called ghosting occurs.

In order to reduce the ghosting, it is effective to provide the cover glass and the filter with an anti-reflection coating. However, providing the anti-reflection coating leads to increased costs.

Japanese Patent Application Laid-Open Publication No. 2021-129625 discloses a camera head in which a pressing portion of an elastic portion presses an optical element formed of a translucent material such as a sapphire glass to fix the optical element in a state being inclined with respect to an optical axis. With this camera head, no consideration is given to reducing ghosting caused by reflected light.

The present inventions aims to provide a low-cost and compact filter device with reduced ghosting.

### SUMMARY OF THE INVENTION

A filter device of an embodiment includes: a filter configured to attenuate light with a predetermined wavelength from emitted light emitted from a cover glass of an eyepiece portion of an endoscope along an optical axis; a frame configured to hold the filter; and a connector configured to hold the frame, and attach the frame to a camera head including an image pickup device configured to receive light passed through the filter. The connector includes an elastic region that presses the filter or the frame to an outer circumference of an effective optical path of the cover glass or to a circumference of the cover glass, in a state where the camera head is attached to the eyepiece portion.

According to the embodiment, it is possible to provide a low-cost and compact filter device with reduced ghosting.

### BRIEF DESCRIPTION OF THE DRAWINGS

Fig. 1 is a configuration diagram of an endoscope system of an embodiment.
Fig. 2 is a partial cross-sectional view of the endoscope system in which a camera head is not attached to an eyepiece portion of an endoscope.
Fig. 3 is a perspective view of a front surface of a filter device of the embodiment.
Fig. 4 is a perspective view of a back surface of the filter device of the embodiment.
Fig. 5 is a partial cross-sectional view of the endoscope system in which the camera head is attached to the eyepiece portion of the endoscope.
Fig. 6 is a partial enlarged view of Fig. 5.
Fig. 7 is a perspective view of a front surface of a filter device of a modified example 1 of the embodiment.
Fig. 8 is a perspective view of a front surface of a filter device of a modified example 2 of the embodiment.
Fig. 9 is a partial cross-sectional view of an endoscope system including the filter device of the modified example 1.
Fig. 10 is a partial cross-sectional view of an endoscope system including the filter device of the modified example 2.
Fig. 11 is a partial cross-sectional view of an endoscope system including a filter device of a modification example 3.

### DETAILED DESCRIPTION OF THE PREFERRED EMBODIMENT(S)

An embodiment will be described below with reference to the drawings. The drawings of the embodiment are schematic. The relationship between thicknesses and widths of respective parts, a ratio of a thickness of a certain part to that of another part, and the like in the drawings are different from the actual ones. The respective drawings include parts in which the relationships and ratios among the dimensions are different. Illustration and reference signs for some constituent elements will be omitted.

### <Configuration of Endoscope System>

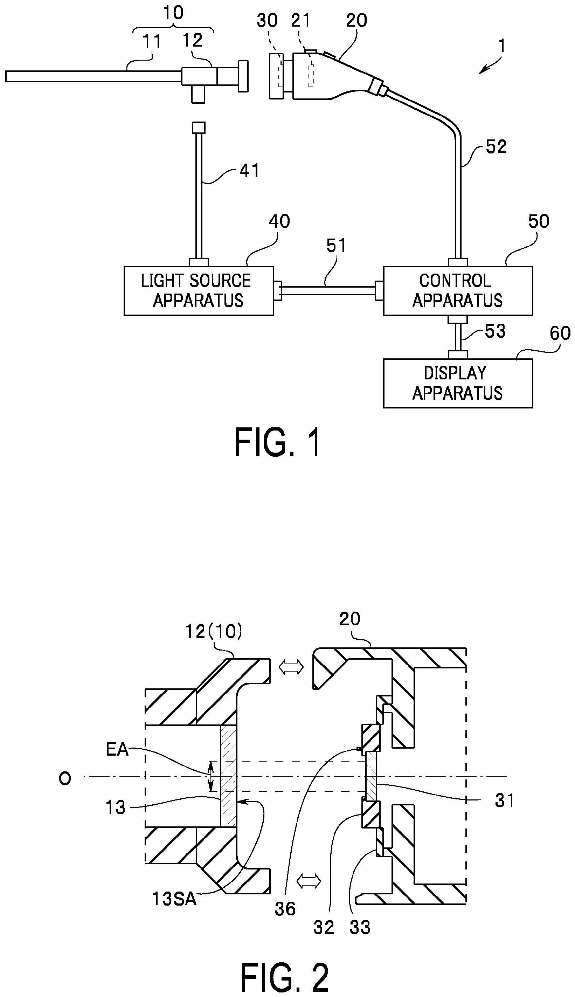

As shown in Fig. 1, an endoscope system 1 of the embodiment includes an endoscope 10, a camera head 20, a filter device 30, a light source apparatus 40, a control apparatus 50, and a display apparatus 60. The endoscope 10 and the light source apparatus 40 are connected to each other by an optical cable 41 that guides illumination light. The camera head 20, the light source apparatus 40, and the display apparatus 60 are connected to the control apparatus 50 by transmission cables 51, 52, and 53, respectively.

The endoscope 10 is a so-called rigid endoscope including an insertion portion 11 and an eyepiece portion 12. The endoscope 10 includes the insertion portion 11 the entirety of which is rigid, or a part of which is flexible and the other part of which is rigid. The insertion portion 11 is inserted into a living body. The insertion portion 11 is provided with an optical system, which includes a plurality of lenses and focuses into an object image.

The eyepiece portion 12 is provided at a proximal end of the insertion portion 11. The eyepiece portion 12 is provided with an optical system that emits, from the eyepiece portion 12 to the outside, the object image, into which the optical system of the insertion portion 11 focuses.

The light source apparatus 40 generates light for illuminating an inside of the living body. The light source apparatus 40 is provided separately from the control apparatus 50, but the light source apparatus 40 may be provided inside the control apparatus 50. The light supplied to the endoscope 10 is emitted from a distal end of the insertion portion 11 to irradiate the inside of the living body. The light irradiated to the inside of the living body and reflected in the living body (object image) is condensed by the optical system in the insertion portion 11.

The camera head 20 includes an image pickup device 21. The camera head 20 is detachably attached to the eyepiece portion 12 of the endoscope 10. The image pickup device 21 coverts emitted light emitted from the eyepiece portion 12 of the endoscope 10 along an optical axis O into an electrical signal.

A housing of the eyepiece portion 12 and a housing of the camera head 20 are formed of metal such as aluminum alloy or a rigid resin such as an epoxy resin.

The display apparatus 60 is a display using liquid crystal or the like, and displays an observation image based on a video signal from the control apparatus 50.

The control apparatus 50 includes a CPU (central processing unit) or the like, and controls the entire endoscope system 1.

As shown in Fig. 2, a cover glass 13 is disposed at a proximal end opening of the eyepiece portion 12 of the endoscope 10. The filter device 30 is attached to the housing of the camera head 20. The filter device 30 includes a filter 31, a frame 32 that holds the filter 31, and a connector 33 that holds the frame 32. The connector 33 attaches the frame 32 to the camera head 20 including the image pickup device 21 which receives light passed through the filter 31.

The endoscope system 1 performs a fluorescent observation of cancer cells. That is, the fluorescent observation is performed by taking advantage of the fact that cancer cells specifically absorb 5-aminolevulinic acid and biosynthesize PpIX (protoporphyrin IX) which generates fluorescence. Specifically, when PpIX is irradiated with blue excitation light with a wavelength of 380 nm, PpIX generates red fluorescence with a wavelength of 380 nm.

The filter 31 is a so-called band-pass filter that blocks most of excitation light with a wavelength of 380 nm from the emitted light emitted through the cover glass 13 of the eyepiece portion 12 of the endoscope 10, but passes most of light other than the excitation light, including fluorescence with a wavelength of 380 nm. Therefore, the endoscope system 1 can display high-quality images with reduced effects of the excitation light on the display apparatus 60 during an endoscopic surgery.

Note that the filter 31 may attenuate the excitation light by at least 70% or more, and in particular may attenuate the excitation light by 99.9% or more.

When the endoscope system 1 performs a laser surgery, the filter 31 blocks laser light with a wavelength of 532 nm, which is effective for vaporizing, resecting, and cutting a tissue, or laser light with a wavelength of 1064 nm, which enhances a coagulation effect.

When the camera head 20 is attached to the endoscope 10 (eyepiece portion 12), after the eyepiece portion 12 is fitted into the camera head 20, the camera head 20 is fixed by being rotated. When the filter device 30 is attached to the camera head 20, after a fitting portion 35, which extends from an outer circumference of the connector 33 of the filter device 30, is fitted into the camera head 20, then the fitting portion 35 is rotated, to fix the filter device 30 is fixed.

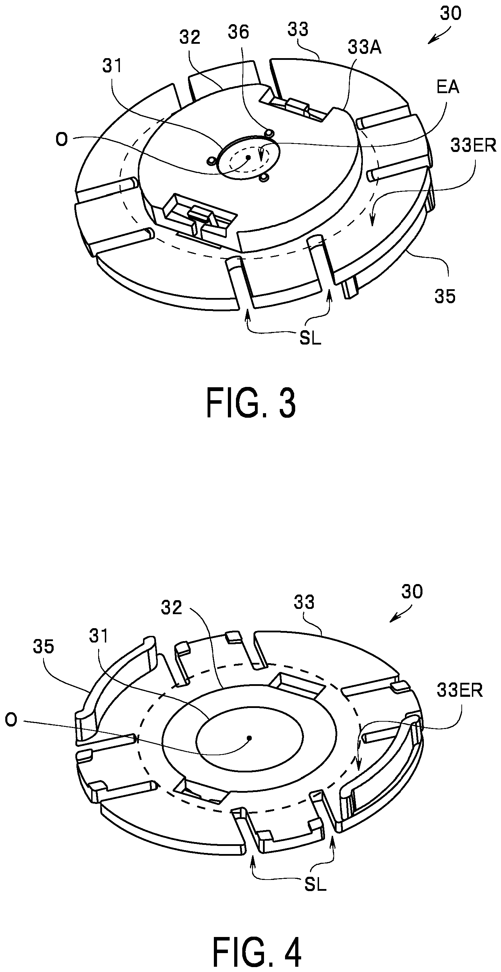

As shown in Fig. 3, the filter device 30 includes, on the substantially circular frame 32 that holds the filter 31, three protrusions 36 protruding in an optical axis direction. The three protrusions 36 are arranged at rotational symmetry positions about the optical axis O. Heights, that is, dimensions in the optical axis direction, of the three protrusions 36 are the same.

The connector 33 of the filter device 30 is formed of a rigid material, and includes a plurality of slits SL arranged in rotational symmetry about the optical axis O. Therefore, an outer circumference region of the connector 33 configures an elastic region 33ER that elastically deforms in the optical axis direction. The elastic region 33ER may be made thin, or may be formed of a flexible material.

The endoscope 10 (eyepiece portion 12) and the camera head 20, and the camera head 20 and the filter device 30 are each detachably attached. Therefore, the filter 31 is not always arranged parallel to the cover glass 13. As already described, if the filter 31 is not arranged parallel to the cover glass 13, ghosting occurs.

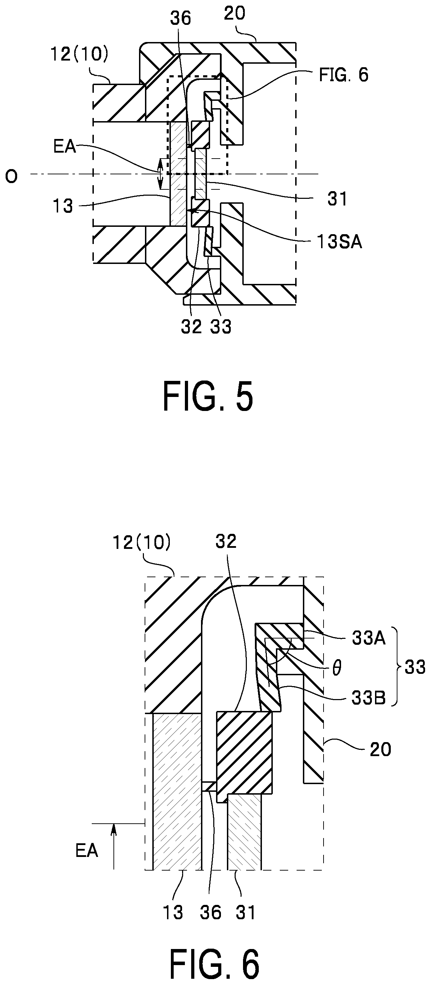

As shown in Fig. 5 and Fig. 6, when the camera head 20 is attached to the eyepiece portion 12, the elastic region 33ER of the connector 33 presses the three protrusions 36 of the frame 32 to an outer circumference of an effective optical path EA of the cover glass 13.

That is, as shown in Fig. 6, the frame 32 (filter 31) moves in a direction toward the image pickup device 21 due to the deformation of the elastic region 33ER in a state where the camera head 20 is attached to the eyepiece portion 12, compared to a state where the camera head 20 is not attached to the eyepiece portion 12.

A distance between the filter 31 and the cover glass 13 is defined by the heights of the protrusions 36. In addition, by the three protrusions 36 arranged on a circumference, a parallelism between the filter 31 and the cover glass 13 is ensured.

In other words, the connector 33 includes a first member 33A, which is the fitting portion 35 for attaching the frame 32 to the housing of the camera head 20 and parallel to the optical axis O, and a second member 33B, which is the elastic region 33ER continuously provided to the first member 33A and perpendicular to the optical axis O. When the camera head 20 is attached to the eyepiece portion 12, an angle θ formed by the first member 33A and the second member 33B changes from a right angle to an acute angle as shown in Fig. 6.

In the filter device 30, the filter 31 is arranged parallel to the cover glass 13 with certainty, so that no ghosting occurs. That is, according to the embodiment, it is possible to provide the low-cost and compact filter device 30 with reduced ghosting, and the endoscope system 1 that includes the low-cost and compact filter device 30 with reduced ghosting.

The filter device 30 is inexpensive because it does not require an anti-reflection coating. Furthermore, in the filter device 30, when the camera head 20 is attached to the eyepiece portion 12, the angle θ formed by the first member 33A and the second member 33B changes from the right angle to the acute angle, so that the dimension in the optical axis direction becomes shorter.

Note that in order that the filter 31 is arranged parallel to the cover glass 13 with certainty, the number of the protrusions 36 may be three or more.

### <Modified Examples>

Filter devices 30A to 30E of the endoscope system 1 of modified examples are similar to the filter device 30 of the embodiment and have the same effects. Therefore, in the following description, constituent elements having the same functions as those of the filter device 30 are denoted by the same reference signs as those of the filter device 30 and the descriptions thereof will be omitted.

### <Modified Example 1>

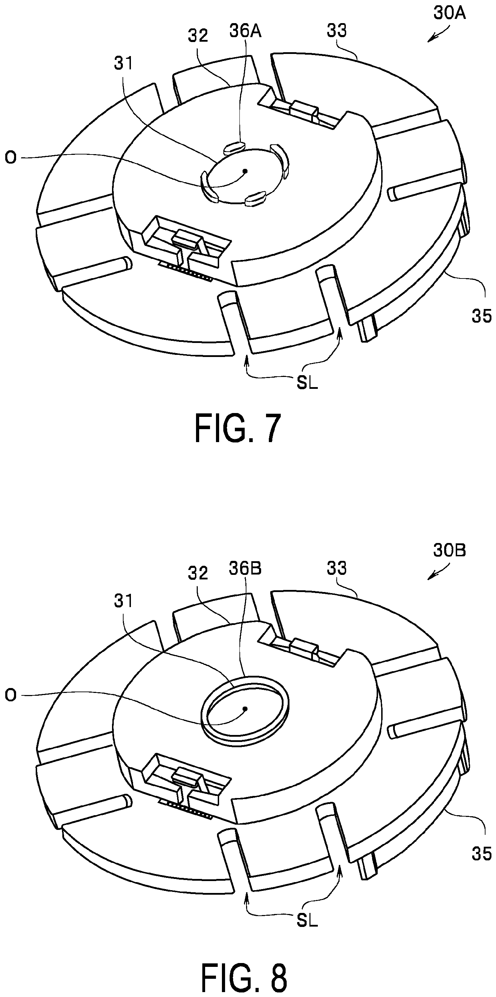

In the filter device 30A of the present modified example shown in Fig. 7, the frame 32 includes a plurality of arc-shaped protrusions 36A arranged in rotational symmetry about the optical axis direction and having the same heights.

If the frame 32 includes the two or more arc-shaped protrusions 36A symmetrically arranged about the optical axis O, the filter 31 can be arranged parallel to the cover glass 13 with certainty.

### <Modified Example 2>

The frame 32 of the filter device 30B of the present modified example shown in Fig. 8 includes an annular protrusion 36B that has a uniform height in the optical axis direction and surrounds the effective optical path EA of the cover glass 13.

If the frame 32 includes the one annular protrusion 36B, the filter 31 can be arranged parallel to the cover glass 13 with certainty.

### <Modified Example 3>

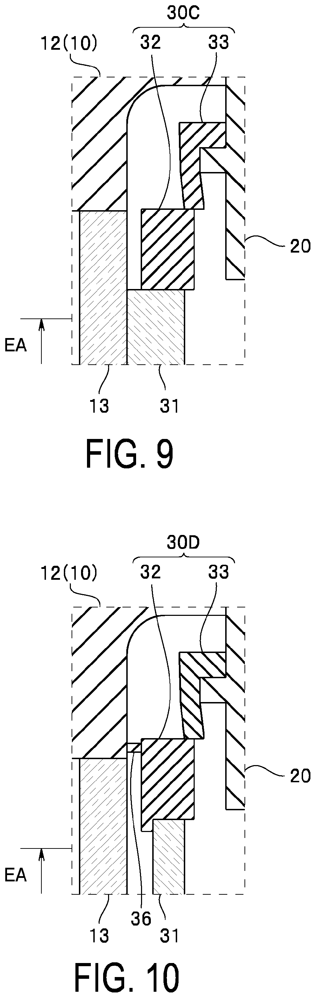

In the filter device 30C of the present modified example shown in Fig. 9, the elastic region 33ER presses the filter 31 to the outer circumference of the effective optical path EA of the cover glass 13 in a state where the camera head 20 is attached to the eyepiece portion 12.

### <Modified Example 4>

In the filter device 30D of the present modified example shown in Fig. 10, the elastic region 33ER presses the protrusion 36 to a circumference of the cover glass 13 in a state where the camera head 20 is attached to the eyepiece portion 12. The protrusion 36 has the same configuration as the protrusion 36 in the embodiment, the modified example 1, or the modified example 2.

### <Modified Example 5>

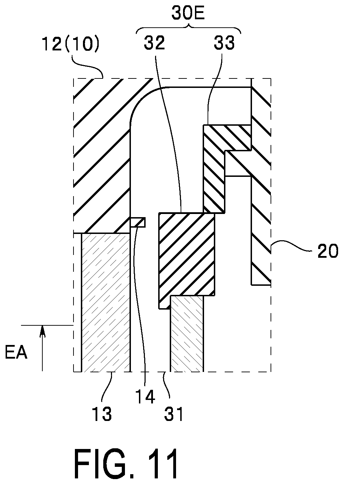

In the filter device 30E of the present modified example shown in Fig. 11, at least one protrusion 14 that protrudes in the optical axis direction of the eyepiece portion 12 comes into contact with an outer circumference of an effective optical path EA of the filter 31 in a state where the camera head 20 is attached to the eyepiece portion 12.

The effective optical path EA of the filter 31 and the effective optical path EA of the cover glass 13 are substantially the same size. The protrusion 14 has the same configuration as the protrusion 36 in the embodiment, the modified example 1, or the modified example 2.
10: endoscope
11: insertion portion
12: eyepiece portion
13: cover glass
14: protrusion
20: camera head
21: image pickup device
30, 30A to 30E: filter device
31: filter
32: frame
33: connector
33ER: elastic region
35: fitting portion
36, 36A, 36B: protrusion
40: light source apparatus
41: optical cable
50: control apparatus
51, 52: transmission cable
60: display apparatus

## Claims

1. A filter device (30, 30A, 30B, 30C, 30D, 30E) comprising:
a filter (31) configured to attenuate light with a predetermined wavelength from emitted light emitted through a cover glass (13) of an eyepiece portion (12) of an endoscope (10) along an optical axis;
a frame (32) configured to hold the filter (31); and
a connector (33) configured to hold the frame (32), and attach the frame (32) to a camera head (20) including an image pickup device (21) configured to receive light passed through the filter (31), the connector (33) including an elastic region (33ER) that presses the filter (31) or the frame (32) to an outer circumference of an effective optical path (EA) of the cover glass (13) or to a circumference of the cover glass (13), in a state where the camera head (20) is attached to the eyepiece portion (12).

2. The filter device (30, 30A, 30B, 30C, 30D, 30E) according to claim 1, wherein the frame (32) includes at least one protrusion (36, 36A, 36B) that protrudes in an optical axis direction.

3. The filter device (30, 30A, 30B) according to claim 2, wherein the protrusion (36, 36A, 36B) comes into contact with the outer circumference of the effective optical path (EA) of the cover glass (13).

4. The filter device (30) according to claim 2, wherein the frame (32) includes three or more protrusions (36) that are arranged in rotational symmetry in the optical axis direction and have the same heights.

5. The filter device (30A) according to claim 2, wherein the frame (32) includes a plurality of arc-shaped protrusions (36A) that are arranged in rotational symmetry in the optical axis direction and have the same heights.

6. The filter device (30B) according to claim 2, wherein the frame (32) includes the protrusion (36B) having an annular shape, the protrusion (36B) having a uniform height in the optical axis direction, and surrounding the effective optical path (EA).

7. The filter device (30, 30A, 30B) according to claim 1, wherein:
the elastic region (33ER) includes a plurality of slits (SL) arranged in rotational symmetry about the optical axis; and
the frame (32) and the connector (33) are each formed of a rigid material.

8. The filter device (30, 30A, 30B) according to claim 1, wherein the frame (32) moves in a direction toward the image pickup device (21) in a state where the camera head (20) is attached to the eyepiece portion (12), compared to a state where the camera head (20) is not attached to the eyepiece portion (12).

9. The filter device (30, 30A, 30B) according to claim 1, wherein:
the connector (33) includes a first member (33A) for attaching the frame (32) to the camera head (20) and parallel to the optical axis, and a second member (33B) continuously provided to the first member (33A) and perpendicular to the optical axis, the second member being configured to hold the filter (31); and
in a state where the camera head (20) is attached to the eyepiece portion (12), the second member (33B) forms an acute angle with the first member (33A).

10. The filter device (30E) according to claim 1, wherein at least one protrusion (14) that protrudes in an optical axis direction of the eyepiece portion (12) comes into contact with an outer circumference of an effective optical path (EA) of the filter (31).
